# EUROPEAN PATENT SPECIFICATION

(11) **EP 4 301 320 B1**
(45) Date of publication and mention of the grant of the patent: **07.05.2025**
(21) Application number: 22710562.4
(22) Date of filing: 04.03.2022
(51) Int. Cl.: A61K 8/24, A61K 8/25, A61K 8/64, A61Q 11/00

(54) **ORAL CARE COMPOSITION**
MUNDPFLEGEZUSAMMENSETZUNG
COMPOSITION DE SOINS BUCCO-DENTAIRES

(30) Priority: 05.03.2021 WO PCT/CN2021/079388; 13.04.2021 EP 21168018
(43) Date of publication of application: 10.01.2024
(73) Proprietor: Unilever IP Holdings B.V., 3013 AL Rotterdam (NL); Unilever Global IP Limited, Wirral, Merseyside CH62 4ZD (GB)
(72) Inventor: LIU, Weining, 6708 WH Wageningen (NL); WANG, Weichong, 6708 WH Wageningen (NL)
(74) Representative: Tansley, Sally Elizabeth
(86) International application number: PCT/EP2022/055495
(87) International publication number: WO 2022/184874

(56) References cited:
- WO-A1-2015/176867
- WO-A1-2021/023547
- CN-A- 110 074 988
- US-B1- 6 652 875

## Description

### Technical Field of the Invention

The present invention relates to an oral care composition. In particular, the present invention relates to an oral care composition comprising calcium silicate, casein phosphopeptide and a phosphate source that results in reduced sensitivity and/or remineralization of teeth. The invention also relates to the use of such compositions for remineralizing and/or reducing sensitivity of teeth of an individual.

### Background of the Invention

Teeth comprise dentin overlaid with an outer layer of enamel. Teeth are under constant attack from chemical and physical forces, including bacteria-derived acids and mechanical wear, resulting in demineralization and weakening of enamel and the underlying dentin.

Tooth hypersensitivity is a temporary induced pain sensation that affects up to 20% of the adult population. It is associated with tooth demineralization and the loss of either enamel or cementum to expose underlying dentin. The dentin of the tooth generally contains channels, called tubules, which provide for an osmotic flow between the inner pulp region of the tooth and the outer root surfaces. The cause of tooth hypersensitivity may be related to demineralization giving rise to increased exposure of tubules and permeability of the dentine. The most common causes of demineralization of the enamel or dentine are attrition, abrasion, gingival recession and erosion. When root surfaces are exposed, dentinal tubules are also exposed.

The currently accepted theory for tooth hypersensitivity is the hydrodynamic theory, based on the belief that open exposed dentinal tubules allow fluid flow through the tubules. This flow excites the nerve endings in the dental pulp. Clinical replica of sensitive teeth viewed in a SEM (scanning electron microscopy) reveal varying numbers of open or partially occluded dentinal tubules.

Efforts have been made over the years to treat tooth hypersensitivity. One approach is to reduce the excitability of the nerve in a sensitive tooth by using "nerve-depolarising agents" comprising strontium ions, potassium salts such as potassium nitrate, potassium bicarbonate, potassium chloride and the like. These nerve-depolarising agents function by interfering with neural transduction of the pain stimulus to make the nerve less sensitive.

Another approach is to use "tubule blocking agents" that fully or partially occlude tubules such as polystyrene beads, apatite, polyacrylic acid, mineral hectorite clay and the like. These tubule blocking agents function by physically blocking the exposed ends of the dentinal tubules, thereby reducing dentinal fluid movement and reducing the irritation associated with the shear stress described by the hydrodynamic theory.

WO 2021/023547A1 (BRASSELER GMBH & CO KG GEB) relates to a preparation that contains: at least one calcium compound, selected from calcium phosphate, calcium fluorides and calcium fluorophosphates and hydroxyl derivatives and carbonate derivatives of said calcium salts, calcium hydroxides and calcium oxides, said calcium compound being precipitated with at least one protein component, selected from proteins and protein hydrolysates; and at least one crosslinking agent for the protein component and/or non-set cement.

A variety of products have been developed in an attempt to treat tooth hypersensitivity and/or enhance tooth remineralization. But there still remains a need to develop an oral care composition which is more effective in remineralizing teeth and/or occluding dentinal tubules to reduce tooth sensitivity.

### Summary of the Invention

In a first aspect, the present invention is directed to an oral care composition comprising:
a) calcium silicate;
b) casein phosphopeptide; and
c) a phosphate source;
wherein the phosphate source comprises trisodium phosphate, monosodium dihydrogen phosphate, disodium hydrogen phosphate, ammonium phosphate, diammonium hydrogen phosphate, ammonium dihydrogen phosphate, tripotassium phosphate, monopotassium dihydrogen phosphate, dipotassium hydrogen phosphate or a mixture thereof.

In a second aspect, the present invention is directed to a packaged oral care product comprising the oral care composition of the first aspect of this invention.

In a third aspect, the present invention is directed to a method for remineralizing and/or reducing sensitivity of teeth of an individual comprising the step of applying the oral care composition of any embodiment of the first aspect to at least one surface of the teeth of an individual. The method is preferably for non-therapeutic benefits.

In a fourth aspect, the present invention is directed to non-therapeutic use of the oral care composition of any embodiment of the first aspect for remineralizing and/or reducing sensitivity of teeth of an individual.

In a fifth aspect, the present invention is directed to use of the oral care composition of any embodiment of the first aspect in the manufacture of a medicament in remineralizing and/or reducing sensitivity of an individual.

All other aspects of the present invention will more readily become apparent upon considering the detailed description and examples which follow.

### Detailed Description

Except in the examples, or where otherwise explicitly indicated, all numbers in this description indicating amounts of material or conditions of reaction, physical properties of materials and/or use may optionally be understood as modified by the word "about".

All amounts are by weight of the final oral care composition, unless otherwise specified. It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

"Soluble" and "insoluble" for the purpose of the present invention means the solubility of a source (e.g., like phosphate source) in water at 25°C and atmospheric pressure. "Soluble" means a source that dissolves in water to give a solution with a concentration of at least 0.1 moles per litre. "Insoluble" means a source that dissolves in water to give a solution with a concentration of less than 0.001 moles per litre. "Slightly soluble", therefore, is defined to mean a source that dissolves in water to give a solution with a concentration of greater than 0.001 moles per litre and less than 0.1 moles per litre.

Now it has been found unexpectedly that an oral care composition comprising calcium silicate, casein phosphopeptide and a phosphate source can efficiently occlude dentinal tubules to provide excellent tubule blockage efficacy to reduce tooth sensitivity. In addition, such oral care composition can also provide enhanced tooth remineralization efficacy. "Remineralization" for the purpose of the present invention means *in situ* (i.e. in the oral cavity) generation of calcium phosphate on teeth (including layers on teeth from 10 nm to 20 microns, and preferably from 75 nm to 10 microns, and most preferably, from 150 nm to 5 microns thick including all ranges subsumed therein) to reduce the likelihood of tooth sensitivity, tooth decay, regenerate enamel and/or improve the appearance of teeth by whitening through the generation of such new calcium phosphate.

The calcium silicate suitable for use in the present invention comprises calcium oxide (CaO) and silica (SiO₂) and is made up of varying proportions of CaO and SiO₂. Preferably, the calcium silicate is insoluble, present as the composite material calcium oxide-silica (CaO-SiO₂), which is described, for example, in international patent application published as WO 2008/01517(Unilever)**.**

For a calcium silicate composite material, the atom ratio of calcium to silicon (Ca:Si) may be from 1:30 to 3:1. The Ca:Si ratio is preferably 1:20 to 3:1, and more preferably, from 1:10 to 3:1, and most preferably, from about 1:7 to 3:1. The calcium silicate may comprise mono-calcium silicate, bi-calcium silicate, or tricalcium silicate.

The calcium silicate may be crystalline or amorphous or even mesoporous. Preferably the calcium silicate is at least partially crystalline. When the calcium silicate is partially crystalline, it comprises calcium (Ca) and silicon (Si) in an atom ratio (Ca:Si) from 1:3 to 3:1, more preferably from 1:1.5 to 1.5:1 and most preferably from 1:1.1 to 1.1:1. It is particularly preferable that the calcium silicate is fully crystalline. The presence of crystallinity may be confirmed, for example, by x-ray diffraction. An example of the calcium silicate suitable for use in the present invention is commercially available under the trade name NYAD^{®} M9000 from NYCO Minerals, Inc.

Preferably, the calcium silicate of the present invention is not hydrated. The calcium silicate which is not hydrated may comprise other components in addition to calcium oxide and silica, such as metal cations, anions and the like. It is preferable that the calcium silicate comprises calcium oxide, silica in an amount of at least 70% by weight of the calcium silicate, more preferably at least 80%, more preferably still at least 90%, even more preferably at least 95% and most preferably the calcium silicate consist of (or at least consist essentially of) calcium oxide, silica.

The calcium silicate may be hydrated. When the calcium silicate is hydrated, the calcium silicate hydrate comprises at least calcium oxide, silica and water. Compared with conventional calcium silicate which are not hydrated, the calcium silicate hydrate comprises the water of hydration in an amount of at least 5% by weight of the calcium silicate hydrate, preferably at least 10%, more preferably at least 15%, even more preferably at least 20% and most preferably at least 25%. The water content is typically no greater than 50% by weight of the calcium silicate hydrate, more preferably no greater than 40%, even more preferably no greater than 35% and most preferably no greater than 30%. "Water of hydration" for the purpose of the present invention means water chemically combined with a substance in a way that it can be removed by heating without substantially changing the chemical composition of the substance. In particular, water which could only be removed when heated above 200°C. The water loss is measured using thermo gravimetric analysis (TGA) with a Netzsch TG instrument. The TGA is conducted under an N₂ atmosphere with heating rate of 10 degree/min in the range of 30 to 900°C.

It is preferable that the calcium silicate of the present invention is particulate which allows for maximum surface area for contact with dental tissue. Preferably the calcium silicate used in this invention has a particle size from 100 nm to 50 microns, more preferably from 500 nm to 30 microns, more preferably still from 700 nm to 20 microns and most preferably from 1 micron to 15 microns. "Particle size" for the purpose of the present invention means D50 particle size. The D50 particle size of a particulate material is the particle size diameter at which 50 wt% of the particles are larger in diameter and 50 wt% are smaller in diameter.

Typically, the oral care composition comprises from 0.1 to 80% by weight of the calcium silicate, more preferably from 0.2 to 50% and most preferably from 1 to 30%, based on total weight of the oral care composition and including all ranges subsumed therein.

The casein phosphopeptide suitable for use in the present invention comprises multiple phosphoseryl residues, which may be made synthetically by chemical synthesis or genetic engineering or extracted from naturally occurring materials. Preferably, the casein phosphopeptide may be obtained by hydrolyzing or digesting (either chemical or proteolytic) a protein or by tryptic digestion of casein or other phosphor-acid rich proteins such as phosphitin, or by chemical or recombinant synthesis. It is preferred that the casein phospopeptide comprises the core sequence -Ser(P)-Ser(P)-Ser(P)-Glu-Glu-, where Ser(P) is a phosphoseryl residue.

The oral care composition typically comprises the casein phosphopeptide in an amount of from 0.01 to 15% by weight of the composition, more preferably from 0.05 to 10% and most preferably from 0.1 to 5%, based on total weight of the oral care composition and including all ranges subsumed therein.

The oral care composition preferably comprises the calcium silicate and the casein phosphopeptide in a weight ratio from 5:1 to 100:1, more preferably from 10:1 to 50:1 and most preferably from 10:1 to 35:1, including all ratios subsumed therein.

The phosphate source used in this invention is limited only to the extent that the same may be used in a composition suitable for use in the mouth. Preferably the phosphate source is water soluble. The phosphate source suitable for use in this invention include trisodium phosphate, monosodium dihydrogen phosphate, disodium hydrogen phosphate, ammonium phosphate, diammonium hydrogen phosphate, ammonium dihydrogen phosphate, tripotassium phosphate, monopotassium dihydrogen phosphate, dipotassium hydrogen phosphate, mixtures thereof or the like.

The oral care composition preferably comprises the phosphate source in an amount of from 0.01 to 4% by weight of the composition, preferably from 0.05 to 2%, more preferably from 0.1 to 2% and most preferably from 0.1 to 1%, based on total weight of the oral care composition and including all ranges subsumed therein. In a preferred embodiment, the phosphate source is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein. In another preferred embodiment, the phosphate source is or at least comprises monosodium dihydrogen phosphate.

Preferably, the oral care composition is an aqueous composition. The water content is at least 1.5% by weight of the oral care composition, preferably at least 5%, more preferably at least 10%. It is preferable that the water content is from 1.5 to 95% by weight of the oral care composition, more preferably from 5 to 90% and most preferably from 10 to 90%, based on total weight of the oral care composition and including all ranges subsumed therein.

Typically, the oral care composition has a pH from 5.5 to 10.5, more preferably from 6.5 to 10, and most preferably from 7.0 to 9.0. The pH of oral care composition is measured when 5 parts by weight of the composition is uniformly dispersed and/or dissolved in 20 parts by weight pure water at 25°C. In particular, the pH may be measured by manually mixing 5 g oral care composition with 20 mL water for 30 s, then immediately testing the pH with indicator or a pH meter.

The oral care composition of the present invention may also comprise a physiologically acceptable carrier. The carrier preferably comprises at least surfactant, thickener, humectant or a combination thereof.

Preferably the oral care composition comprises a surfactant. Preferably the composition comprises at least 0.01% surfactant by weight of the composition, more preferably at least 0.1% and most preferably from 0.5 to 7%. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate, sodium coco sulfate, cocamidopropyl betaine, sodium methyl cocoyl taurate or mixtures thereof.

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, xanthan gum and/or sodium carboxymethyl cellulose and/or a Carbomer is/are preferred. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

In another especially preferred embodiment, the thickener is xanthan gum.

Thickener typically makes up from 0.01 to about 10%, more preferably from 0.1 to 9%, and most preferably, from 0.1 to 5% by weight of the oral care composition, based on total weight of the composition and including all ranges subsumed therein.

When the oral care composition of this invention is a toothpaste or gel, the same typically has a viscosity from about 30,000 to 180,000 centipoise, and preferably, from 60,000 to 170,000 centipoise, and most preferably, from 65,000 to 165,000 centipoise, taken at room temperature (25°C) with a Brookfield Viscometer, Spindle No.93/94 and at a speed of 5 rpm for 1 minute.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the oral care composition. More preferably, the carrier humectant makes up from 25 to 80%, and most preferably, from 30 to 60% by weight of the composition, based on total weight of the composition and including all ranges subsumed therein.

The oral care composition may further comprise benefit agents that are typically delivered to human teeth and/or the oral cavity including the gums to enhance or improve a characteristic of those dental tissues. The benefit agents are present in the oral care composition in addition to the calcium silicate, the casein phosphopeptide and the phosphate source that are included in the composition.

Typically the benefit agent is selected from optical agents, biomineralization agents, adsorbing agents, anti-microbial agents, gum health agents, desensitizing agents, anti-calculus agents, freshness agents and mixtures thereof.

Optical agents may be selected from one or more of whitening agents and pearlescent agents. When the optical agent is a whitening agent, it is preferable that the whitening agent is a particulate whitening agent having a refractive index of from 1.9 to 4.0. The refractive index is quoted at a temperature of 25°C and a wavelength of 589 nm. Suitable materials to provide such a high refractive index are metal compounds and preferred are compounds where the metal is selected from zinc (Zn), titanium (Ti), zirconium (Zr) or a mixture thereof. Preferably, the metal compound is (or at least comprises) a metal oxide such as titanium dioxide (TiO₂), zinc oxide (ZnO), zirconium dioxide (ZrO₂) or a mixture thereof. Preferably, the particulate whitening agent is a composite particle. "Composite particle", as used herein, means a particle comprising a first component core and a second component coating wherein the core and coating are composed of different materials. Particularly suitable core materials are metal compounds and preferred are compounds where the metal is selected from zinc (Zn), titanium (Ti), zirconium (Zr) or a mixture thereof. Preferably, the metal compound is (or at least comprises) a metal oxide such as titanium dioxide (TiO₂), zinc oxide (ZnO), zirconium dioxide (ZrO₂) or a mixture thereof. The second component coating comprises material suitable to adhere to tooth enamel, dentin or both. Typically the coating material comprises the element calcium, and optionally, other metals like potassium, sodium, aluminium, magnesium as well as mixtures thereof whereby such optional metals are provided as, for example, sulphates, lactates, oxides, carbonates or silicates. Optionally, the coating material may be aluminium oxide or silica. In a preferred embodiment, the second component coating can comprise, for example, calcium phosphate, calcium gluconate, calcium oxide, calcium lactate, calcium carbonate, calcium hydroxide, calcium sulphate, calcium carboxymethyl cellulose, calcium alginate, calcium salts of citric acid, calcium silicate, mixture thereof or the like. Calcium silicate is particularly preferred. In an especially preferred embodiment, the particulate whitening agent is titanium dioxide coated with calcium silicate.

In another preferred embodiment, the whitening agent is a dye or a pigment or a combination of the two. The pigment according to the invention is a shade/material which is insoluble in the relevant medium, at the relevant temperature. This is in contrast to dyes which are soluble. The term "relevant medium", as used herein, refers to human saliva, the liquid medium in which the composition is used, at the temperature of the oral cavity during brushing of the teeth, i.e. up to 37°C. The relevant medium may also be water and the relevant temperature to be 25°C. Preferred pigment or dye for use in the present invention is violet or blue having a hue angle, h, in the CIELAB system of from 220 to 320 degrees, preferably from 250 to 290 degrees. When the whitening agent is a pigment, it is preferable that the pigment is violet or blue selected from one or more of those listed in the Colour Index International as pigment blue 1 through to pigment blue 83 and pigment violet 1 through to pigment violet 56. Other suitable pigments are pigment ultramarine blue and ultramarine violet. While the preferred pigment is blue or violet, the same effect may be achieved through mixing pigments outside of this hue angle range.

For example, such a hue angle may also be obtained by mixing a red and green-blue pigment to yield a blue or violet shaded pigment. It is particularly preferred that the pigment is a blue pigment. A preferred class of blue pigments suitable for use in the invention are organic blue pigments such as phthalocyanine blue pigments. It is especially preferred that the pigment is phthalocyanine blue pigment selected from alpha copper phthalocyanines Pigment Blue 15, Pigment Blue 15:1, Pigment Blue 15:2, Pigment Blue 15:3, Pigment Blue 15:4, Pigment Blue 15:6 and mixtures thereof. Most preferably the pigment is Pigment Blue 15:1. A commercially available example is Cosmenyl Blue A4R from Clariant. When the whitening agent is a dye, it is preferable that the dye is a blue dye, more preferably FD&C Blue 1 or Patent Blue V.

In another preferred embodiment, the optical agent is a pearlescent agent. The term "pearlescent", as used herein, means an agent in the form of particles which reflects and partially transmits the incident light. Suitable pearlescent agent for use in the present invention includes mica, bismuth oxychloride or mixtures thereof. Preferably the pearlescent agent is formed by coating one or more metal oxide layers onto natural or synthetic mica flakes. Examples of suitable mica include, for example, muscovite, phlogopite, fluorophlogopite, biotite or mixtures thereof. Examples of suitable metal oxides include, for example, TiO₂, Fe₂O₃, TiFe₂O₅, Fe₃O₄, Cr₂O₃, Al₂O₃, SiO₂, ZrO₂, ZnO, SnO₂, CoO, Co₃O₄, VO₂, V₂O₃, Sn(Sb)O₂, titanium suboxides or mixtures thereof. One example of a preferred pearlescent agent is mica coated with TiO₂.

Biomineralization agents for tooth enamel remineralization may be selected from one or more of calcium sources, biomolecules, proteinaceous materials, amorphous calcium phosphate, α-tricalcium phosphate, β-tricalcium phosphate, calcium silicate, calcium sulfate, calcium carbonate, calcium deficient hydroxyapatite Ca_{10-X}(HPO₄)_{X}(PO₄)_{6-X}(OH)_{2-X}, 0 ≤ x < 1), dicalcium phosphate (CaHPO₄), dicalcium phosphate dihydrate (CaHPO₄·2H₂O), hydroxyapatite (Ca₁₀(PO₄)₆(OH)₂), monocalcium phosphate monohydrate (Ca(H₂PO₄)₂·H₂O), octacalcium phosphate (Ca₈H₂(PO₄)₆·5H₂O) and tetracalcium phosphate (Ca₄(PO₄)₂O).

Adsorbing agents may be selected from one or more of boron nitride, porous silica, charcoal, layered silicate (e.g. montmorillonite, kaoline, clay), graphene and polymethyl methacrylate microsphere.

Anti-microbial agents may be selected from one or more of metal salts where the metal is selected from zinc, copper, silver or a mixture thereof, triclosan, triclosan monophosphate, triclocarban, curcumin, quaternary ammonium compounds, bisbiguanides and long chain tertiary amines, preferably zinc salts including zinc oxide, zinc chloride, zinc acetate, zinc ascorbate, zinc sulphate, zinc nitrate, zinc citrate, zinc lactate, zinc peroxide, zinc fluoride, zinc ammonium sulfate, zinc bromide, zinc iodide, zinc gluconate, zinc tartarate, zinc succinate, zinc formate, zinc phenol sulfonate, zinc salicylate, zinc glycerophosphate or a mixture thereof.

Desensitizing agents may be selected from one or more of potassium citrate, potassium chloride, potassium tartrate, potassium bicarbonate, potassium oxalate, potassium nitrate and strontium salts.

Anti-calculus agents may be selected from one or more of alkali-metal pyrophosphates, hypophosphite-containing polymers, organic phosphonates and phosphocitrates.

Gum health agents may be selected from one or more of anti-inflammatory agents such as ibuprofen, flurbiprofen, aspirin and indomethacin, plaque buffers, biomolecules, proteinaceous materials, vitamins such as Vitamins A,C and E, plant extracts and curcumin.

Freshness agents may be flavours selected from one or more of peppermint, spearmint, menthol, flora oil, clove oil and citrus oil.

The benefit agent is preferably selected from optical agents, biomineralization agents and mixtures thereof. In an especially preferred embodiment, the benefit agent is an optical agent, especially a whitening agent.

The oral care composition of the present invention may comprise a single benefit agent or a mixture of two or more benefit agents. Typically, the benefit agent is present in an amount from 0.1 to 60%, and more preferably, from 0.3 to 40%, and most preferably from 0.5 to 30% by total weight of the oral care composition and including all ranges subsumed therein.

The oral care composition may additionally comprise a fluoride source. Preferred fluoride source includes sodium fluoride, stannous fluoride, potassium fluoride, sodium monofluorophosphate, sodium fluorosilicate, ammonium fluorosilicate, amine fluoride, ammonium fluoride or mixtures thereof. Preferably, the fluoride source is stannous fluoride, sodium fluoride, sodium monofluorophosphate or mixtures thereof. Sodium monofluorophosphate is particularly preferred. The fluoride source may be present at a level from 0.01 to 10%, more preferably from 0.03 to 5% and most preferably from 0.1 to 2% by weight of the oral care composition, based on total weight of the composition and including all ranges subsumed therein.

The oral care composition may additionally comprise abrasives. Preferred abrasives include silicas, aluminas, calcium carbonates, dicalcium phosphates, calcium pyrophosphates, hydroxyapatites, trimetaphosphates, insoluble hexametaphosphates or mixtures thereof, including agglomerated particulate abrasives. Calcium carbonate and silica are particularly preferred, especially silica. The abrasives may be present in the range of from 0.01 to 60%, more preferably from 0.1 to 30%, and most preferably from 1 to 15% by weight of the oral care composition, based on total weight of the composition and including all ranges subsumed therein.

The oral care composition of the present invention may contain a variety of other ingredients which are common in the art to enhance physical properties and performance. These ingredients include preservatives, pH-adjusting agents, sweetening agents, polymeric compounds, buffers and salts to buffer the pH and ionic strength of the compositions, and mixtures thereof. Such ingredients typically and collectively make up less than 20% by weight of the composition, and preferably, from 0.0 to 15% by weight, and most preferably, from 0.01 to 12% by weight of the composition, including all ranges subsumed therein.

In a preferred embodiment, the oral care composition is a monophase composition.

In another preferred embodiment, the oral care composition is a dual-phase composition comprising a calcium phase and a phosphate phase, wherein the calcium silicate is present in the calcium phase and the phosphate source and the casein phosphopeptide are present in the phosphate phase. The two phases are physically separate from one another by being in independent phases. The delivery of the two independent phases to the teeth may be simultaneous or sequential. In a preferred embodiment, the phases are delivered simultaneously.

Typically, the dual-phase composition is delivered by a dual-tube having a first compartment for calcium phase and a second compartment for phosphate phase, which allows for co-extrusion of the two phases.

In a preferred embodiment, such a dual-tube has one of the compartments surrounding the other. In such embodiments, one phase is present as a sheath, surrounding the other phase in the core. In an especially preferred embodiment, the core is the calcium phase and the sheath is the phosphate phase.

In another preferred embodiment, such a dual-tube has the two compartments side by side within the same tube. In such embodiments, the two phases are extruded from the tube as one, such extrusion being termed "contact extrusion". A pump head may be used in such a dual-tube for squeezing the two phases from the tube as one.

The dual-phase oral care composition may be a gel composition that comprises two independent gel phases, the first is the calcium phase and the second is the phosphate phase. The means of delivery may involve a cotton rod, or a tray, onto which the calcium phase and the phosphate phase are applied, prior to the tray being placed in contact with the teeth.

The oral care composition of this invention can be used in a method for remineralizing and/or reducing sensitivity of teeth of an individual. Preferably, the method is non-therapeutic. The oral care composition of this invention may additionally be used for remineralizing and/or reducing sensitivity of teeth of an individual, and/or used in the manufacture of a medicament in remineralizing and/or reducing sensitivity of an individual. Preferably, the use is non-therapeutic.

The oral care composition may be in any form common in the art, preferred forms are dentifrices, tooth pastes, gels, mouthwashes, medicaments, oral films, serums, chewing gums and lozenges. Typically the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area. In liquid mouthwash form the composition may be packaged in a bottle, sachet or other convenient container.

The composition can be effective even when used in an individual's daily oral hygiene routine. For example, the composition may be brushed onto the teeth. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day.

The following examples are provided to facilitate an understanding of the present invention. The examples are not provided to limit the scope of the claims.

### Examples

### Example 1

This example demonstrates the effect of calcium silicate and casein phosphopeptide on blockage of dentinal tubules. All ingredients are expressed by weight percent of the total formulation.

**TABLE 1**

| **Ingredient** | **Samples** | | | |
|---|---|---|---|---|
| | **A** | **1** | **2** | **3** |
| Water | To 100 | To 100 | To 100 | To 100 |
| Sorbitol (70/70) | 50.00 | 50.00 | 50.00 | 50.00 |
| Xanthan gum | 0.50 | 0.50 | 0.50 | 0.50 |
| Glycerine | 19.00 | 19.00 | 19.00 | 19.00 |
| PEG 32 | 0.20 | 0.20 | 0.20 | 0.20 |
| Sodium monofluorophosphate | 1.11 | 1.11 | 1.11 | 1.11 |
| Sodium Saccharin | 0.20 | 0.20 | 0.20 | 0.20 |
| Calcium silicate^{a} | 15.00 | 15.00 | 15.00 | 15.00 |
| Monosodium dihydrogen phosphate | 0.50 | 0.50 | 0.50 | 0.50 |
| Casein phosphopeptide | -- | 0.50 | 1.25 | 2.00 |
| Silica abrasive | 4.00 | 4.00 | 4.00 | 4.00 |
| Thickening silica | 4.00 | 4.00 | 4.00 | 4.00 |
| Sodium lauryl sulfate | 1.80 | 1.80 | 1.80 | 1.80 |
| Fragrance | 0.80 | 0.80 | 0.80 | 0.80 |

| | | | | |
|---|---|---|---|---|
| a. Commercially available crystalline calcium silicate under the trade name NYAD^{®} M9000 from NYCO Minerals, Inc. | | | | |

### Methods

### Brushing protocol

To evaluate the blockage efficacy of dentinal tubules, fresh slurries were prepared by mixing toothpaste with de-ionised (DI) water or sodium phosphate solution for 40 seconds and used immediately.

Human dentine discs were eroded by 6% citric acid for 2 mins, then they were treated with different slurries via brushing following the same protocol. Eight human dentine discs were separated into four groups (n=2). The dentine discs were brushed with the slurry under a tooth brushing machine equipped with toothbrushes. The load of the tooth brushing was 170 g +/-5 g and the automatic brushing operated at a speed of 150 rpm. After brushing for 1 min, the dentine discs were soaked in slurry for 1 min. Then the dentine discs were placed in 50 mL DI water and agitated on a flatbed shaker at 150 rpm for 10 strokes. The discs were then soaked in simulated oral fluid (SOF) for at least 6 hours under the condition of a shaking water bath at 37°C and 60.0 rpm. After that, the dentine discs were brushed with the slurry by machine using the same procedure as in the first step. The brushing was repeated twice for one day, then the dentine discs were kept in SOF overnight (>12 hours) in a shaking water bath at 37°C to mimic oral environment. The dentine samples were characterized by scanning electron microscopy (SEM, Hitachi S-4800, Japan) after 3 days of brushings.

Simulated oral fluid was made by combining the ingredients in Table 2:

**TABLE 2**

| **Ingredient** | **Amount/g** |
|---|---|
| NaCl | 16.07 |
| NaHCO₃ | 0.7 |
| KCI | 0.448 |
| K₂HPO₄*3H₂O | 3.27 |
| MgCl₂*6H₂O | 0.0622 |
| 1M HCl | 40 mL |
| CaCl₂ | 0.1998 |
| Na₂SO₄ | 0.1434 |
| Buffer | Adjust pH to 7.0 |
| Water | Balance to 2 L |

### Scoring Standard for Tubules Blockage

Regardless of the original shape of the dentine discs, a square (with a size of 4mm x 4mm) is selected and one image is captured under 30x magnification. Within this square, five spots (each with a size of 150 µm x 150 µm, one in the middle, and one in every corner) are selected and observed under 1000x magnification. The blockage of tubules is accessed following the standards described in Table 3. The measurement is carried out for the two dentine discs of each test group.

**TABLE 3**

| **Score** | **Tubules Blockage** |
|---|---|
| 0 | All dentinal tubules are open. |
| 1 | <20% of dentinal tubules are fully blocked. |
| 2 | 20 to 50% of dentinal tubules are fully blocked. |
| 3 | 50 to 80% of dentinal tubules are fully blocked. |
| 4 | 80-100% of dentinal tubules are fully blocked. |
| 5 | All dentinal tubules are fully blocked. |

### Results

SEM images of the dentine discs were taken after 3 days of brushings. The images were analyzed and scored. The results are reported in table 4 (error represents standard error for duplicate measurements).

**TABLE 4**

| **Tubules Blockage Score** | **Samples** | | | |
|---|---|---|---|---|
| | **A** | **1** | **2** | **3** |
| 3 days of brushings^{b} | 3.2^{A} ± 0.36 | 4.4^{B} ± 0.27 | 4.5^{B} ± 0.17 | 4.3^{B} ± 0.15 |

| | | | | |
|---|---|---|---|---|
| b. Values with different letter are significantly different (p<0.05) | | | | |

It can be seen from the results that samples comprising additional casein phosphopeptide had significantly better tubule blockage efficacy compared to sample A.

### Example 2

This example demonstrated the effect of calcium source on blockage of dentinal tubules. All ingredients are expressed by weight percent of the total formulation.

**TABLE 5**

| **Ingredient** | **Samples** | | |
|---|---|---|---|
| | **1** | **4** | **5** |
| Water | To 100 | To 100 | To 100 |
| Sorbitol (70/70) | 50.00 | 50.00 | 50.00 |
| Xanthan gum | 0.50 | 0.50 | 0.50 |
| Glycerine | 19.00 | 19.00 | 19.00 |
| PEG 32 | 0.20 | 0.20 | 0.20 |
| Sodium monofluorophosphate | 1.11 | 1.11 | 1.11 |
| Sodium Saccharin | 0.20 | 0.20 | 0.20 |
| Calcium silicate^{a} | 15.00 | -- | -- |
| Bioactive glass^{c} | -- | 15.00 | -- |
| Calcium carbonate^{d} | -- | -- | 15.00 |
| Monosodium dihydrogen phosphate | 0.50 | 0.50 | 0.50 |
| Casein phosphopeptide | 0.50 | 0.50 | 0.50 |
| Silica abrasive | 4.00 | 4.00 | 4.00 |
| Thickening silica | 4.00 | 4.00 | 4.00 |
| Sodium lauryl sulfate | 1.80 | 1.80 | 1.80 |
| Fragrance | 0.80 | 0.80 | 0.80 |

| | | | |
|---|---|---|---|
| c. Commercially available bioactive glass (45S5 bioglass) from Kunshan Huaqiao New Materials Co., Ltd. d. Commercially available calcium carbonate under the trade name OMYACARE S80-QY from Omya Mineral (Anhui Qingyang) Co.,Ltd | | | |

### Methods

The same protocol was used to evaluate the blockage of dentinal tubules as described in Example 1. The dentine samples were brushed twice daily for three days.

### Results

SEM images of the dentine discs were taken after 3 days of brushings. The images were analyzed and scored. The results are reported in table 6 (error represents standard error for duplicate measurements).

**TABLE 6**

| **Tubules Blockage Score** | **Samples** | | |
|---|---|---|---|
| | **1** | **4** | **5** |
| 3 days of brushings^{b} | 4.4^{A} ± 0.27 | 3.4^{B} ± 0.22 | 2.6^{C} ± 0.16 |

Sample 1 comprising calcium silicate showed significantly better tubule blockage efficacy compared to other samples.

## Claims

1. An oral care composition comprising:
a) calcium silicate;
b) casein phosphopeptide; and
c) a phosphate source;
wherein the phosphate source comprises trisodium phosphate, monosodium dihydrogen phosphate, disodium hydrogen phosphate, ammonium phosphate, diammonium hydrogen phosphate, ammonium dihydrogen phosphate, tripotassium phosphate, monopotassium dihydrogen phosphate, dipotassium hydrogen phosphate or a mixture thereof.

2. The oral care composition according to claim 1, wherein the calcium silicate is at least partially crystalline.

3. The oral care composition according to claim 2, wherein the presence of crystallinity of the calcium silicate is confirmed by x-ray diffraction.

4. The oral care composition according to any of the preceding claims, wherein the calcium silicate is fully crystalline.

5. The oral care composition according to any of the preceding claims, wherein the calcium silicate comprises Ca and Si in an atom ratio of from 1:3 to 3 :1, preferably from 1:1.5 to 1.5:1.

6. The oral care composition according to any of the preceding claims, wherein the composition comprises the calcium silicate in an amount of from 0.1 to 80% by weight of the composition, preferably from 0.2 to 50%.

7. The oral care composition according to any of the preceding claims, wherein the casein phosphopeptide comprises the core sequence -Ser(P)-Ser(P)-Ser(P)-Glu-Glu-.

8. The oral care composition according to any of the preceding claims, wherein the composition comprises the casein phosphopeptide in an amount of from 0.01 to 15% by weight of the composition, preferably from 0.05 to 10%.

9. The oral care composition according to any of the preceding claims, wherein the calcium silicate and the casein phosphopeptide are present in a weight ratio ranges from 5:1 to 100:1, preferably from 10:1 to 50:1.

10. The oral care composition according to any of the preceding claims, wherein the composition comprises the phosphate source in an amount of from 0.01 to 4% by weight of the composition, preferably from 0.05 to 2%.

11. The oral care composition according to any of the preceding claims, wherein the composition additionally comprises a benefit agent selected from optical agents, biomineralization agents, adsorbing agents, anti-microbial agents, gum health agents, desensitizing agents, anti-calculus agents, freshness agents and mixtures thereof, preferably the benefit agent is an optical agent, more preferably the benefit agent is a whitening agent.

12. The oral care composition according to any of the preceding claims, wherein the composition is an aqueous composition.

13. The oral care composition according to claim 12, wherein the water content is from 1.5 to 95% by weight of the composition.

14. A method for remineralizing and/or reducing sensitivity of teeth of an individual comprising the step of applying the composition according to any one of claims 1 to 13 to at least one surface of the teeth of the individual.

## Patentansprüche

1. Mundpflegezusammensetzung, umfassend:
a) Calciumsilikat;
b) Caseinphosphopeptid; und
c) eine Phosphatquelle;
wobei die Phosphatquelle umfasst Trinatriumphosphat, Mononatriumdihydrogenphosphat, Dinatriumhydrogenphosphat, Ammoniumphosphat, Diammoniumhydrogenphosphat, Ammoniumdihydrogenphosphat, Trikaliumphosphat, Monokaliumdihydrogenphosphat, Dikaliumhydrogenphosphat oder eine Mischung davon.

2. Mundpflegezusammensetzung nach Anspruch 1, wobei das Calciumsilikat zumindest teilweise kristallin ist.

3. Mundpflegezusammensetzung nach Anspruch 2, wobei das Vorhandensein von Kristallinität des Calciumsilikats durch Röntgenbeugung bestätigt wird.

4. Mundpflegezusammensetzung nach einem der vorhergehenden Ansprüche, wobei das Calciumsilikat vollständig kristallin ist.

5. Mundpflegezusammensetzung nach einem der vorhergehenden Ansprüche, wobei das Calciumsilikat Ca und Si in einem Atomverhältnis von 1:3 bis 3:1, bevorzugt von 1:1,5 bis 1,5:1, umfasst.

6. Mundpflegezusammensetzung nach einem der vorhergehenden Ansprüche, wobei die Zusammensetzung das Calciumsilikat in einer Menge von 0,1 bis 80 Gew.-% der Zusammensetzung, bevorzugt von 0,2 bis 50%, umfasst.

7. Mundpflegezusammensetzung nach einem der vorhergehenden Ansprüche, wobei das Caseinphosphopeptid die Kernsequenz -Ser(P)-Ser(P)-Ser(P)-Glu-Glu-umfasst.

8. Mundpflegezusammensetzung nach einem der vorhergehenden Ansprüche, wobei die Zusammensetzung das Caseinphosphopeptid in einer Menge von 0,01 bis 15 Gewichts-% der Zusammensetzung, bevorzugt von 0,05 bis 10%, umfasst.

9. Mundpflegezusammensetzung nach einem der vorhergehenden Ansprüche, wobei das Calciumsilikat und das Caseinphosphopeptid in einem Gewichtsverhältnis in dem Bereich von 5:1 bis 100:1, bevorzugt von 10:1 bis 50:1, vorhanden sind.

10. Mundpflegezusammensetzung nach einem der vorhergehenden Ansprüche, wobei die Zusammensetzung die Phosphatquelle in einer Menge von 0,01 bis 4 Gewichts-% der Zusammensetzung, bevorzugt von 0,05 bis 2%, umfasst.

11. Mundpflegezusammensetzung nach einem der vorhergehenden Ansprüche, wobei die Zusammensetzung zusätzlich einen Wirkstoff umfasst, der aus optischen Mitteln, Biomineralisierungsmitteln, Adsorptionsmitteln, antimikrobiellen Mitteln, Mitteln für die Zahngesundheit, Desensibilisierungsmitteln, Mitteln gegen Zahnstein, Erfrischungsmitteln und Mischungen davon ausgewählt ist, wobei der Wirkstoff bevorzugt ein optisches Mittel, bevorzugter ein Aufhellungsmittel ist.

12. Mundpflegezusammensetzung nach einem der vorhergehenden Ansprüche, wobei die Zusammensetzung eine wässrige Zusammensetzung ist.

13. Mundpflegezusammensetzung nach Anspruch 12, wobei der Wassergehalt 1,5 bis 95 Gewichts-% der Zusammensetzung beträgt.

14. Verfahren zur Remineralisierung und/oder der Verringerung der Empfindlichkeit der Zähne einer Person, umfassend den Schritt des Auftragens der Zusammensetzung nach irgendeinem der Ansprüche 1 bis 13 auf mindestens eine Oberfläche der Zähne einer Person.

## Revendications

1. Composition de soins bucco-dentaires comprenant:
a) du silicate de calcium;
b) du phosphopeptide de caséine; et
c) une source de phosphate;
où la source de phosphate comprend du phosphate de trisodium, du dihydrogénophosphate de monosodium, de l'hydrogénophosphate de disodium, du phosphate d'ammonium, de l'hydrogénophosphate de diammonium, du dihydrogénophosphate d'ammonium, du phosphate de tripotassium, du dihydrogénophosphate de monopotassium, de l'hydrogénophosphate de dipotassium ou un mélange de ceux-ci.

2. Composition de soins bucco-dentaires selon la revendication 1, où le silicate de calcium est au moins partiellement cristallin.

3. Composition de soins bucco-dentaires selon la revendication 2, où la présence de cristallinité du silicate de calcium est confirmée par diffraction des rayons X.

4. Composition de soins bucco-dentaires selon l'une quelconque des revendications précédentes, où le silicate de calcium est entièrement cristallin.

5. Composition de soins bucco-dentaires selon l'une quelconque des revendications précédentes, où le silicate de calcium comprend Ca et Si dans un rapport atomique de 1:3 à 3:1, de préférence de 1:1,5 à 1,5:1.

6. Composition de soins bucco-dentaires selon l'une quelconque des revendications précédentes, où la composition comprend le silicate de calcium en une quantité de 0,1 à 80 % en poids de la composition, de préférence de 0,2 à 50 %.

7. Composition de soins bucco-dentaires selon l'une quelconque des revendications précédentes, où le phosphopeptide de caséine comprend la séquence centrale -Ser(P)-Ser(P)-Ser(P)-Glu-Glu-.

8. Composition de soins bucco-dentaires selon l'une quelconque des revendications précédentes, où la composition comprend le phosphopeptide de caséine en une quantité de 0,01 à 15 % en poids de la composition, de préférence de 0,05 à 10 %.

9. Composition de soins bucco-dentaires selon l'une quelconque des revendications précédentes, où le silicate de calcium et le phosphopeptide de caséine sont présents dans un rapport pondéral allant de 5:1 à 100:1, de préférence de 10:1 à 50:1.

10. Composition de soins bucco-dentaires selon l'une quelconque des revendications précédentes, où la composition comprend la source de phosphate en une quantité de 0,01 à 4 % en poids de la composition, de préférence de 0,05 à 2 %.

11. Composition de soins bucco-dentaires selon l'une quelconque des revendications précédentes, où la composition comprend en outre un agent bénéfique choisi parmi les agents optiques, les agents de biominéralisation, les agents adsorbants, les agents antimicrobiens, les agents de santé des gencives, les agents désensibilisants, les agents antitartre, les agents de fraîcheur et leurs mélanges, de préférence l'agent bénéfique est un agent optique, de préférence encore l'agent bénéfique est un agent de blanchiment.

12. Composition de soins bucco-dentaires selon l'une quelconque des revendications précédentes, où la composition est une composition aqueuse.

13. Composition de soins bucco-dentaires selon la revendication 12, où la teneur en eau est de 1,5 à 95 % en poids de la composition.

14. Procédé pour reminéraliser et/ou réduire la sensibilité des dents d'un individu comprenant l'étape d'application de la composition selon l'une quelconque des revendications 1 à 13 à au moins une surface des dents de l'individu.
